# EUROPEAN PATENT SPECIFICATION

(11) **EP 2 555 785 B1**
(45) Date of publication and mention of the grant of the patent: **06.04.2016**
(21) Application number: 11721384.3
(22) Date of filing: 07.04.2011
(51) Int. Cl.: A61K 35/74, A61P 11/00

(54) **USE OF STREPTOCOCCUS SALIVARIUS IN THE TREATMENT OF CHRONIC INFECTIONS OF THE RESPIRATORY TRACT**
VERWENDUNG VON STREPTOCOCCUS SALIVARIUS ZUR BEHANDLUNG VON ATEMWEGSINFEKTIONEN
UTILISATION DE STREPTOCOCCUS SALIVARIUS POUR LE TRAITEMENT D'INFECTIONS DE LA VOIE RESPIRATOIRE

(30) Priority: 07.04.2010 IT RM20100163
(43) Date of publication of application: 13.02.2013
(73) Proprietor: D.M.G. Italia Srl, 00040 Pomezia (RM) (IT)
(72) Inventor: STEFANI, Stefania, I-95124 Catania (CT) (IT); TIBERI, Licia, I-00040 Pomezia (RM) (IT); SANTAGATI, Maria., I-95124 Catania (CT) (IT)
(74) Representative: Sarpi, Maurizio
(86) International application number: PCT/IT2011/000104
(87) International publication number: WO 2011/125086

(56) References cited:
- WO-A1-2004/072272
- WALLS TONY ET AL: "Bacteriocin-like inhibitory substance (BLIS) production by the normal flora of the nasopharynx: potential to protect against otitis media?", JOURNAL OF MEDICAL MICROBIOLOGY SEP 2003 LNKD- PUBMED:12909662, vol. 52, no. Pt 9, September 2003 (2003-09), pages 829-833, XP002603368, ISSN: 0022-2615

## Description

The present invention provides a new microbial strain of the species *Streptococcus salivarius* for use in the treatment of inflammatory processes with or without infectious etiology. A further object of the present invention are compositions comprising said strain and uses thereof.

### STATE OF THE ART

Many of the ENT (Ear, Nose and Throat) diseases may originate from a fungal or bacterial infection in the upper tracts of the respiratory system; examples of such infections are some forms of otitis, sinusitis and/or nasal poliposis: usually the treatment of such forms is performed by using topical or oral antibiotics or anti-inflammatory agents.

Recently clinical studies have demonstrated that the administration of streptococci such as *Streptococcus mitis, Streptococcus sanguinis, Streptococcus oralis* in the form of spray to patients affected by Acute Otitis Media (AOM) interferes and/or inhibits the growth of pathogenic microorganisms responsible of the disease. However, these species of microorganisms have the serious disadvantage to be classified as potentially pathogenic species.

The international patent application WO2004/072272 describes the use of a biologically pure culture of *S. salivarius,* isolated from patients oral cavity, in antibacterial compositions for the treatment of otitis media.

Walls et al. disclose that *Streptococcus salivarius* produces bacteriocin-like inhibitory substances (BLIS) which inhibit the activity of acute otitis media pathogens in the nasopharingeal floraof children and suggest that due to their low pathogenicity *S. salivarius* should be incorporated into trial of bacteriotherapy of recurrent AOM (Walls et al., 2003).

Recently Power et al. (Power et al. Eur J Clin Microbiol Infect Dis. 2008, 1261-3) have carried out preliminary studies on a group of children affected by AOM by administrating orally a pediatric composition comprising the strain *S. salivarius* K12. This strain has been previously used as a probiotic for oral hygiene and anti-halitosis.

The study carried out by Power and colleagues has revealed that only in a small percentage of the treated patients, the strain *S. salivarius* K12 has colonized the upper respiratory tract causing an improvement in symptoms of the disease being treated. The low capacity of the so far isolated strains of *S. salivarius* to colonize the upper respiratory tract makes less efficient their use in the adjuvant therapy against infections of the respiratory tracts.

So it was so strongly felt the need to isolate new non pathogenic strains that in addition to bactericide activity provide high capacity to colonize the respiratory tract.

### SUMMARY OF THE INVENTION

The inventors have succeeded in isolating from the nasopharynx of a healthy voluntary, a new bacterial strain belonging to the species *S. salivarius* deposited at the Institute Deutsche Sammlung von Mikroorganismen und Zellkulturen (DSMZ) under the filing number DSM 23307 in date 4 February 2010.

The inventors, by *in vitro* experiments, show that this specific strain of *Streptococcus salivarius* is characterized by:
i) high inhibitory activity towards *S. pneumoniae,* stable in various culture conditions (BAC and TSYE);
ii) inhibitory activity towards particularly virulent and antibiotics multi-resistant serotypes responsible of invasive infections such as strain *S. pneumoniae* 19A;
iii) inhibitory activity towards *S. pyogenes* M-type 1;
iv) high adhesion capacity to the cells HEp-2 (epithelial cells of human carcinoma of the larynx) up to 57%;
v) absence of virulence genes;
vi) complete sensitivity to antibiotics.

Adhesion capacity of this strain to cells HEp-2, together with the properties not belonging to a pathogenic or potentially pathogenic species and producing bacteriocins able to inhibit the growth of *S. pneumoniae* and *S. pyogenes,* makes the strain of *Streptococcus salivarius* selected by the inventors and any other strain of *Streptococcus salivarius* with such features particularly suitable for treating bacterial and/or fungal infections of the upper respiratory tract. The utility of such organisms, that can be administered by pharmaceutical compositions, lies in their ability to colonize the respiratory tracts competing pathogenic species. It is therefore clear that adhesion ability of the administered strains to the HEp-2 type cells plays a key role for the efficacy of the same. The pattern of adhesion *in vitro* on cells derived from upper respiratory tract provides the adhesion and the retention of the bacteriocins producing strains.

Therefore, object of the present invention is a bacterial strain belonging to the *Streptococcus salivarius* species characterized by the ability to adhere to HEp-2 cells.

A further object of the invention is said bacterial strain as above defined and compositions comprising it for treating infections and/or inflammations of the upper respiratory tract.

Compositions comprising said bacterial strain and one or more carriers and/or diluents and/or excipients are object of the invention as well.

The advantages, features and the use modes of the present invention are evident from the following detailed description in some embodiments, presented as an example and without limitation.

### DETAILED DESCRIPTION OF THE INVENTION

The present invention provides a new bacterial strain belonging to the species *Streptococcus salivarius* isolated by the inventors from the nosepharynx of a human voluntary subject; the strain has been identified by phenotypic and genotypic analysis.

The inventors have analyzed several nasal and pharyngeal swabs and several bacterial species have been isolated therefrom, but in one case only it has been isolated and selected a strain with the desired characteristics. The strain has a typical morphology of the *S. salivarius* species with a round shape of the colony and size of 1-2 mm in diameter, with entire and smooth margins. The bacterial strain can be grown on culture medium "Mitis salivarius" at 35°C, preferably in presence of 5% CO₂. The strain is able to adhere to HEp-2 cells and to inhibit the growth of the pathogen *S. pneumoniae* by bacteriocins production.

The strain has been called *Streptococcus* salivarius 24 5MBc and submitted in date 4 February 2010 at the Institute Deutsche Sammlung von Mikroorganismen und Zellkulturen (DSMZ) GmbH, Braunschweig, Germany, under the filing number DSM 23307.

As already previously described the ability to adhere to HEp-2 cells makes this strain and even other strains belonging to the species *Streptococcus salivarius* having such feature particularly suitable for treating infections and/or inflammations of the upper respiratory tract, preferably for treating infections causing diseases such as acute otitis media, recurrence otitis media, nasal polyposis, sinusitis.

In the present description are defined as upper respiratory tracts the nasal and paranasal cavities, the pharynx, the larynx.

Object of the present invention are also compositions comprising strains of *Streptococcus salivarius* as above defined and one or more carriers, diluents and/or excipients.

Said compositions preferably comprise the bacterial strain *Streptococcus salivarius* filing number DSM 23307.

Bacteria can be in suspension, freeze-dryed or inactivated, provided they are not killed. The preparation of the compositions of the invention can then be implemented by freeze-drying of bacterial cultures, mixing freeze-dryed both in suspension with water or with further suitable excipients and optionally with addition of further active principles.

The amount of bacteria in said composition is preferably in the range between 10³ and 10¹⁰ CFU for each gram of composition.

Examples of excipients that can be used in such compositions are: rubber, xanthan, carboxylmethyl cellulose, silicone, Vaseline, white soft paraffin, magnesium stearate, maltodextrin, mannitol, starch, glucose, glycerine, propylene glycol, and similar.

Said compositions may include also carriers idoneous to improve the bioavailability, the stability and the endurance of the microrganism.

Said composition may comprise carriers to further improve the adhesion of the microorganism adhesion on the mucosal surface such as the EG56 polymer (Bis-Methoxy PEG-13 PEG-438/PPG-110 SMDI Copolymer), a heat-sensitive polymer able to increase the viscosity and thus the adhesiveness by increasing the temperature or Gantrex (PVM/MA Copolimer).

Said compositions may be in any form considered by the expert of the technical field suitable to be administered topically, orally, or through the respiratory tract.

For administration through the respiratory tract in the present description it has to be intended nasal or by inhalation administration.

Examples of suitable pharmaceutical forms are cream, lotion, gel, ointment, solution, suspension, emulsion, capsule, tablet, powder, granules, sprays, drops.

Preferably compositions may be formulated to be administered through the respiratory tract in a nebulizer, with or without propellants.

Such compositions can be prepared according to techniques and protocols known to the expert of the technical field. Said composition may even contain anti-inflammatory agents such as 18-beta glycyrrhetinic acid.

Object of the present invention are the compositions above described useful for treating infections of the upper respiratory tract, preferably for treating infections causing diseases such as acute otitis media, recurrence otitis media, nasal polyposis, sinusitis.

### EXAMPLES

### Collection of nasal and pharyngeal swabs from patients

Thirty one children aged between 10 and 12 years have been involved in this study. Children who had one, few or any AOM episode have been selected. Patients who received antibiotics in the previous two weeks, had an operation on the upper respiratory tract or with anatomic abnormalities of the respiratory tract have been excluded.

A nasal and pharyngeal swab has been collected respectively from the nostrils and the mouth of each patient with a cotton wool soaked in sterile calcium alginate.

### Microbiological test

In order to highlight the presence of bacterial flora of nasal and pharyngeal swab samples were collected as above described, all samples have been plated onto Mitis Salivarius agar (Difco), a selective medium for streptococci, and onto "chocolate agar" (Columbia Agar Base, OXOID) containing 5% horse blood in order to determine bacterial microflora.

Cultures have been incubated for 18 hours at 37°C in presence of 5% CO₂ and atmospheric pressure. All strains have been frozen at -70°C in "Brain heart infusion broth" (OXOID) with 20% glycerol.

### BLIS (Bacteriocin-like inhibitory substance) test

Each colony morphologically distinct and isolated, obtained from the growth of bacteria as described above has been assayed for the ability to inhibit the most representative strains causing otitis: *S. pyogenes* 2812A, *S. pneumoniae* 11ATN, *H. influenzae* 3ATF, *S. aureus, E. coli, P. aeruginosa, S. salivarius* ATCC13419, *M. catarrhalis.* The ability to inhibit pathogen strains has been assayed by the "BLIS test" as originally developed by Walls et al. (Med microbial 52 (2003)). Assays have been performed by using two different media: Trypticase Soy Yeast Extract Calcium Agar (TSYCa) + 2% Yeast Extract and Blood agar + calcium carbonate (BACa). Results have shown that the strain of *Streptococcus salivarius* identified by filing number DSM 23307 is able to inhibit the growth of *S. pneumoniae* both in TSYCa and BACa medium. Furthermore, it has been evaluated the ability of strain *S. salivarius* DSM 23307 to inhibit particularly virulent and multi-resistant strains of *S. pneumoniae* 19A and *S. pyogenes* M-type 1.

### Analysis of virulence genes

In *S. salivarius* DSM 23307 the presence of virulence genes particularly diffuse in streptococci such as sag A, smez-2 and speB, respectively responsible of the production of the toxin streptolisin S, the mitogenic exotoxin and the eritrogenic exotoxin. The assays have been performed by PCR and hybridization with specific probes.

Results have shown the absence of such virulence genes.

### Adhesion test

To perform the test cells HEp-2 (ATCC CCL 23) have been cultured in essential minimal Eagle media (EMEM) (Invitrogen, Carlsbad, CA). The media was added with 10% bovine serum (FBS), penicillin (100 U/ml) and streptomycin (100 µg/ml). *Streptococcus salivarius* bacteria DSM23307 before being used in the adhesion assay have been grown for 16-18 hours in a 5 ml Todd Hewitt media. Bacteria density has been adjusted according to spectrophotometer readings in order to have a range of density between 10⁵ and 10⁶ CFU/ml before the test. The adhesion test has been performed in HEp-2 cells as described in Benga L. et al.

### Stability test

Stability tests have been performed by incubating the strain *Streptococcus salivarius* DSM 23307 for 18 hours at pH 8.0 in "Tryptic Soy" (TSB), Todd Hewitt and Brain Heart Infusion (BHI) media.

### Results

### Identification of isolated strain

In the table 3 below are identified the species to which belong the strain isolated from the analyzed nasal and pharyngeal swabs:

**Table 3**

| Bacteriocins producer | Molecular identification |
|---|---|
| 3A-TF (1) | *S. mitis* |
| 3A-TF (3) | *S. salivarius* |
| 8A-TF | *S. mitis* |
| 11A-TF (2) | *S. mitis* |
| 14A-TF (2) | *S. salivarius* |
| 14A-TF (4) | *S. salivarius* |
| 15A-TF | *L. cremoris* |
| 19A-TF (1) | *S. sanguis* |
| 21A-TF (3) | *S. mitis* |
| 24A-TF (4) | *S. salivarius* |
| 25A-TF | *L. cremoris* |
| 25A-TN (2) | *S. salivarius* |
| 26A-TF (1) | *S. mitis* |
| 25A-TF (2) | *S. mitis* |

### Identification and characterization of strain Streptococcus salivarius DSM 23307

*Streptococcus salivarius* DSM 23307 has been isolated from the nosepharynx of a human subject. The strain growths on a "Mitis salivarius" medium at 35 °C, 5% CO₂, having the typical morphology of *S. salivarius* species.
Colony shape and size: round, 1-2 mm diameter.
Edge: continuous, smooth.
Colour: Blue.

Grown on Columbia agar with 5% horse blood at 37°C, 5% CO₂ the strain is not haemolytic and has the following morphology
Colony shape and size: round, 1-2 mm diameter.
Edge: continuous, smooth.
Colour: White.

*Streptococcus salivarius* DSM 23307 strain has been analyzed by the commercial kit for the identification of streptococci API 20 Strep. After 24 hours incubation, according to the manufacturer's instruction, has resulted code 5070451, corresponding to the species *Streptococcus salivarius.*

Results obtained by API 20 Strep
Acetoin production: positive
Hydrolisis: negative
β-glucosidase: positive
Pirrolinodil arilamidase: negative α-galactosidase: negative
β-glucuronidase: positive
Alcaline phosphatase: positive
Leucin arilamidase: positive
Arginine dihydrolase: negative
Ribose: negative
L-arabinose: negative
Mannitol: negative
Sorbitol: negative
Lactose: positive
Trealose: positive
Inuline: negative
Raffinose: negative
Glycogen: negative
B-hemolysis: negative

16S and sodA gene sequence analysis have demonstrated that the identified strain belongs to the species *S. salivarius* (99.8% identity).

### Activity of S. salivarius DSM 23307

| Incubation | *S. pyogenes* 2812A M-type 1 | *S. pyogenes* SF370 M-type 1 | *S. pneumoniae* 11A-TN | *H. influenzae* 3A-TF | *S. aureux* | *B. catarrhalis* |
|---|---|---|---|---|---|---|
| BACa | - | - | + | - | - | - |
| TSYE | + | + | + | - | - | - |

### Adhesion experiment

Adhesion assays have demonstrated that the *Streptococcus salivarius* DSM 23307 strain has an excellent ability to adhere to HEp-2 cells, up to 57%, interfering with the adhesion of opportunistic bacteria and fungi.

### Formulations

*1. Streptococcus salivarius* DSM 23307, saline.
2. *Streptococcus salivarius* DSM 23307, EG56 polymer, xanthan, carboxymethyl-cellulose, saline.
3. *Streptococcus salivarius* DSM2 3307, silicone, Vaseline, white soft paraffin, magnesium stearate.
*4. Streptococcus salivarius* DSM 23307, maltodextrin, mannitol, 18 beta-glycyrrhetinic acid, starch.
*5. Streptococcus salivarius* DSM 23307, glucose, deionized water.
*6. Streptococcus salivarius* DSM 23307, propylene glycol and/or glycerine.

In conclusion, the present invention provides a new bacterial strain belonging to the species *Streptococcus salivarius* having biological features making it the one and different from other patented strains indicated for the treatment of the above referred infections.

In particular, the strain *Streptococcus salivarius* DSM 23307 of the present invention inhibit even *S. pneumoniae* (the main pathogenic agent of AOM) in different culturing conditions (BACa and TSYE) and *S. pyogenes* (TSYE).

This feature differentiates it from other described strains belonging to *S. salivarius* such as *S. salivarius* 30 which has inhibitory ability only towards *S. pyogenes* in the two BACa and TSYE media, expanding its range of action only in assays carried out in TSYE.

Furthermore, surprisingly as demonstrated by BLIS tests results, the strain *Streptococcus salivarius* DSM 23307 of the present invention inhibits even the important pathogens such as *S. pneumoniae* 19A and *S. pyogenes* M-type 1, which are frequently isolated from the upper respiratory tracts.

Finally, *S. salivarius* DSM 23307, shows some biological features, such as sensitivity to antibiotics, absence of virulence genes, and adhesive ability up to 57%, which make it the one, well characterized and distinguishable from other *S. salivarius* strains, in particular *S. salivarius* 30. 1.

### BIBLIOGRAPHY

1. Power DA, Burton JP, Chilcott CN, Dawes PJ, Tagg JR. Preliminary investigations of the colonisation of upper respiratory tract tissues of infants using a paediatric formulation of the oral probiotic Streptococcus salivarius K12. Eur J Clin Microbiol Infect Dis. 2008 Dec;27(12):1261-3.
2. Walls T, Power D, Tagg J. Bacteriocin-like inhibitory substance (BLIS) production by the normal flora of the nasopharynx: potential to protect against otitis media? J Med Microbiol. 2003 Sep;52(Pt 9):829-33.
3. Benga L, Goethe R, Rohde M, Valentin-Weigand P. Non-encapsulated strains reveal novel insights in invasion and survival of Streptococcus suis in epithelial cells. Cell Microbiol. 2004 Sep;6(9):867-81.
4. Santagati et al. ESCMID 2010 - Vienna. Poster P1295, p.130.

## Claims

1. An isolated bacterial strain belonging to the species *S. salivarius* deposited at the Deutsche Sammlung von Mikroorganismen und Zellkulturen GmbH, Braunschweig, Germany, under accession number DSM 23307.

2. The bacterial strain according to claim 1 for use in the treatment of infections and/or inflammation of upper respiratory tract.

3. The bacterial strain according to claim 1 for use in the treatment of infections of upper respiratory tract, preferably for treating infections causing diseases such as acute otitis media, recurrent otitis media, sinusitis, nasal polyposis.

4. The bacterial strain according to claim 1 wherein the bacteria are in suspension, freeze-dryed or inactivated, provided they are not killed.

5. Composition comprising the bacterial strain according to claim 1 for use in the treatment of infections and/or inflammation of upper respiratory tract.

6. Composition comprising the bacterial strain according to claim 1 for use in the treatment of infections of upper respiratory tract, preferably for treating infections causing diseases such as acute otitis media, recurrent otitis media, sinusitis, nasal polyposis.

7. Composition for use according to claim 5 or 6 implemented by freeze-drying of bacterial culture, by mixing freeze-dryed bacteria either in suspension with water or with further suitable excipients, and optionally with addition of further active principles.

8. Composition for use according to any claim from 5 to 7 wherein the amount of bacteria is preferably in the range between 10³ and 10¹⁰ CFU for each gram of composition.

9. Composition for use according to any claim from 5 to 8 comprising one or more pharmaceutically acceptable excipients, aromatizing agents or carriers.

10. Composition for use according to claim 9 wherein the used excipients are: rubber, xanthan, carboxylmethyl cellulose, silicone, Vaseline, white soft paraffin, magnesium stearate, maltodextrin, mannitol, starch, glucose, glycerine, propylene glycol, and equivalent molecules.

11. Composition for use according to claim 9 wherein the used carriers are idoneous to improve the bioavalibility, the stability and the endurance of the microrganism.

12. Composition for use according to claim 9 wherein the used carriers improve the adhesion of the microrganism on the mucosal surface such as the Bis-Methoxy PEG-13 PEG-438/PPG-110 SMDI copolymer.

13. Composition for use according to any claim from 5 to 8 comprising anti-inflammatory agents such as 18-beta glycyrrhetinic acid.

14. Composition for use according to any claim from 5 to 13 **characterized in** being in any form suitable to be administered topically, orally or through the respiratory tract.

15. Composition for use according to claim 14 **characterized in** being in the pharmaceutical form of cream, lotion, gel, ointment, solution, suspension, emulsion, capsule, tablet, powder, granules, sprays, drops.

16. Composition for use according to claim 15 **characterized in** being formulated to be administered through the respiratory tract by a nebulizer, with or without propellants.

## Patentansprüche

1. Isolierter Bakterienstamm, der zur Spezies S. salivarius gehört und bei der Deutschen Sammlung für Mikroorganismen und Zellkulturen GmbH, Braunschweig, Deutschland, unter der Zugangsnummer DSM 23307 hinterlegt wurde.

2. Bakterienstamm nach Anspruch 1 zur Verwendung zur Behandlung von Infektionen und/oder einer Entzündung des oberen Respirationstrakts.

3. Bakterienstamm nach Anspruch 1 zur Verwendung bei der Behandlung von Infektionen des oberen Respirationstrakts, bevorzugt zur Behandlung von Infektionen, die Erkrankungen wie eine akute Mittelohrentzündung, eine rezidivierende Mittelohrentzündung, eine Sinusitis oder eine nasale Polypose verursachen.

4. Bakterienstamm nach Anspruch 1, wobei die Bakterien in Suspension, gefriergetrocknet oder inaktiviert vorliegen, vorausgesetzt, dass sie nicht abgetötet sind.

5. Zusammensetzung, umfassend den Bakterienstamm nach Anspruch 1 zur Verwendung zur Behandlung von Infektionen und/oder einer Entzündung des oberen Respirationstrakts.

6. Zusammensetzung, umfassend den Bakterienstamm nach Anspruch 1 zur Verwendung zur Behandlung von Infektionen des oberen Respirationstrakts, bevorzugt zur Behandlung von Infektionen, welche Erkrankungen wie eine akute Mittelohrentzündung, eine rezidivierende Mittelohrentzündung, eine Sinusitis, oder eine nasale Polypose verursachen.

7. Zusammensetzung zur Verwendung nach Anspruch 5 oder 6, implementiert durch Gefriertrocknung einer Bakterienkultur durch Vermischen der gefriergetrockneten Bakterien, sowohl in Suspension mit Wasser oder mit anderen geeigneten Exzipienten, als auch wahlweise mit Zugabe weiterer aktiver Prinzipien.

8. Zusammensetzung zur Verwendung nach einem der Ansprüche 5-7, wobei die Bakterienmenge bevorzugt in einem Bereich von 10³-10¹⁰ CFU pro Gramm Zusammensetzung liegt.

9. Zusammensetzung zur Verwendung nach einem der Ansprüche 5-8, umfassend ein oder mehrere pharmazeutisch wirksame Exzipienten, aromatisierende Mittel oder Träger.

10. Zusammensetzung zur Verwendung nach Anspruch 9, wobei folgende Exzipienten verwendet werden: Gummi, Xanthan, Carboxylmethylcellulose, Silikon, Vaseline, weißes Weichparaffin, Magnesiumstearat, Maltodextrin, Mannitol, Stärke, Glukose, Glycerin, Propylenglycol und äquivalente Moleküle.

11. Zusammensetzung zur Verwendung nach Anspruch 1, wobei die verwendeten Träger geeignet sind, um die Bioverfügbarkeit, die Stabilität und das Überleben des Mikroorganismus zu verbessern.

12. Zusammensetzung zur Verwendung nach Anspruch 7, wobei die verwendeten Träger die Adhäsion des Mikroorganismus an die Schleimhautoberfläche verbessern und die Träger beispielsweise Bis-methoxy PEG-13 PEG-438/PPG-110-SMDI-Copolymer sind.

13. Zusammensetzung zur Verwendung nach einem der Ansprüche 5-8, umfassend entzündungshemmende Mittel wie 18-Beta-glycyrrhetinsäure.

14. Zusammensetzung zur Verwendung nach einem der Ansprüche 5-13, **dadurch gekennzeichnet, dass** sie in irgendeiner Form vorliegt, die geeignet ist um topisch, oral oder über den Respirationstrakt aufgenommen zu werden.

15. Zusammensetzung zur Verwendung nach Anspruch 14, **dadurch gekennzeichnet, dass** sie in der pharmazeutischen Form einer Creme, einer Lotion, eines Gels, einer Salbe, einer Lösung, einer Suspension, einer Emulsion, einer Kapsel, einer Tablette, eines Pulvers, von Granulaten, Sprays oder Drops vorliegt.

16. Zusammensetzung zur Verwendung nach Anspruch 15, **dadurch gekennzeichnet, dass** sie formuliert wurde, um über den Respirationstrakt mit einem Vernebler, mit oder ohne Treibmittel, verabreicht zu werden.

## Revendications

1. Souche bactérienne isolée appartenant à l'espèce S. salivarius déposée auprès du Deutsche Sammlung von Mikroorganismen und Zellkulturen GmbH, Braunschweig, Allemagne, sous le numéro d'accès DSM 23307.

2. Souche bactérienne selon la revendication 1, pour l'utilisation dans le traitement d'infections et/ou d'inflammation des voies respiratoires supérieures.

3. Souche bactérienne selon la revendication 1, pour l'utilisation dans le traitement d'infections des voies respiratoires supérieures, de préférence pour le traitement d'infections à l'origine de maladies telles que l'otite moyenne aiguë, l'otite moyenne récurrente, la sinusite, la polypose nasale.

4. Souche bactérienne selon la revendication 1, dans laquelle les bactéries sont en suspension, lyophilisées ou inactivées, pourvu qu'elles ne soient pas tuées.

5. Composition comprenant la souche bactérienne selon la revendication 1, pour l'utilisation dans le traitement d'infections et/ou d'inflammation des voies respiratoires supérieures.

6. Composition comprenant la souche bactérienne selon la revendication 1, pour l'utilisation dans le traitement d'infections des voies respiratoires supérieures, de préférence pour le traitement d'infections à l'origine de maladies telles que l'otite moyenne aiguë, l'otite moyenne récurrente, la sinusite, la polypose nasale.

7. Composition pour l'utilisation selon la revendication 5 ou 6, mise en oeuvre par lyophilisation de culture bactérienne, par mélange de bactéries lyophilisées soit en suspension avec de l'eau soit avec d'autres excipients appropriés, et éventuellement avec l'ajout d'autres principes actifs.

8. Composition pour l'utilisation selon l'une quelconque des revendications 5 à 7, dans laquelle la quantité de bactéries est de préférence dans la plage entre 10³ et 10¹⁰ CFU pour chaque gramme de composition.

9. Composition pour l'utilisation selon l'une quelconque des revendications 5 à 8, comprenant un ou plusieurs excipients, agents aromatisants ou supports pharmaceutiquement acceptables.

10. Composition pour l'utilisation selon la revendication 9, dans laquelle les excipients utilisés sont : caoutchouc, xanthan, carboxyméthylcellulose, silicone, vaseline, paraffine molle blanche, stéarate de magnésium, maltodextrine, mannitol, amidon, glucose, glycérine, propylène glycol et molécules équivalentes.

11. Composition pour l'utilisation selon la revendication 9, dans laquelle les supports utilisés sont appropriés pour améliorer la biodisponibilité, la stabilité et l'endurance du microorganisme.

12. Composition pour l'utilisation selon la revendication 9, dans laquelle les supports utilisés améliorent l'adhérence du microorganisme sur la surface muqueuse comme le copolymère Bis-Méthoxy PEG-13 PEG-438/PPG-110 SMDI.

13. Composition pour l'utilisation selon l'une quelconque des revendications 5 à 8, comprenant des agents anti-inflammatoires tels que l'acide 18 beta-glycyrrhénitique.

14. Composition pour l'utilisation selon l'une quelconque des revendications 5 à 13, **caractérisée en ce qu'**elle est sous n'importe quelle forme adaptée pour être administrée par voie topique, par voie orale ou à travers les voies respiratoires.

15. Composition pour l'utilisation selon la revendication 14, dans laquelle **caractérisée en ce qu'**elle est sous la forme pharmaceutique de crème, lotion, gel, onguent, solution, suspension, émulsion, capsule, comprimé, poudre, granulés, vaporisateurs ou gouttes.

16. Composition pour l'utilisation selon la revendication 15, **caractérisée en ce qu'**elle est formulée pour être administrée à travers les voies respiratoires avec un nébuliseur, avec ou sans propulseurs.
